Europäisches Patentamt

(19) **European Patent Office**

Office européen des brevets

(11) Publication number: **0 025 248**
**B1**

(12) **EUROPEAN PATENT SPECIFICATION**

(45) Date of publication of patent specification: 16.11.83

(21) Application number: 80200812.8

(22) Date of filing: 29.08.80

(51) Int. Cl.³: **A 61 B 6/02**, A 61 B 6/06, G 21 K 1/02

(54) Device for determining local absorption differences in an object.

(30) Priority: 05.09.79 NL 7906634

(43) Date of publication of application:
18.03.81 Bulletin 81/11

(45) Publication of the grant of the patent:
16.11.83 Bulletin 83/46

(84) Designated Contracting States:
DE FR GB NL

(56) References cited:
DE - C - 964 017
GB - A - 2 004 448
GB - A - 2 007 457
US - A - 4 051 379
US - A - 4 093 863
US - A - 4 096 391
US - A - 4 153 839

(73) Proprietor: N.V. Philips' Gloeilampenfabrieken
Groenewoudseweg 1
NL-5621 BA Eindhoven (NL)

(72) Inventor: Botden, Peter Jacobus Matheus
c/o INT. OCTROOIBUREAU B.V. Prof. Holstlaan 6
NL-5656 AA Eindhoven (NL)

(74) Representative: Rensen, Jan Geert et al,
Internationaal Octrooibureau B.V. Prof. Holstlaan
6
NL-5656 AA Eindhoven (NL)

Courier Press, Leamington Spa, England.

Device for determining local absorption differences in an object

The invention relates to a device for determining local absorption differences in an object section, comprising an X-ray source for generating a flat, fan-shaped X-ray beam and an X-ray detector in the general form of an arc of a circle having as its centre the X-ray source, the X-ray detector being directed radially towards the X-ray source and including a plurality of lamina-shaped collimating elements which also are directed radially towards the X-ray.

Such a device is known from United States Patent Specification 4,093,863 in which the X-ray source, the X-ray detector and a scattered radiation collimator are connected to a rotatable support which has a central aperture for accommodating an object to be examined. The X-ray source is arranged on one side of the central aperture and the X-ray detector and the scattered radiation collimator are arranged on the other side thereof. During an examination, the rotatable support is rotated about the central aperture, so that an object arranged in this aperture is irradiated from different directions. Radiation transmitted by the object is measured by means of the X-ray detector which comprises a large number of individual elemental ionisation chambers which are arranged on an arc of a circle and which are defined by lamina-shaped electrodes which are directed towards the X-ray source. Collimator plates of the scattered radiation collimator are arranged along the respective lines of projection, of the lamina-shaped electrodes towards the X-ray source and are thus directed, like the electrodes, towards the X-ray source.

In US 4,096,391 a device is described in which a collimator is provided with elements which are directed towards the X-ray source and which have the shape of a trapezium with the longer side facing the X-ray source. The need for such a collimator here is given by the fact that use is made of a flat homogeneous detector so that without the collimator radiation scattered in the object and the patient support, would significantly degrade the image forming process. The need for such a collimator is avoided in the device as described in US 4,093,863 by using a detector collimator of which the cells are oriented towards the X-ray source.

The described known devices have a drawback in that an apparent change in the sensitivity of the detector is liable to occur during an examination, thus introducing measurement errors which give rise to disturbing errors in the calculation of the density distribution of the examined section of the object. When use is made of an X-ray source in the form of, for example, a rotary anode X-ray tube, the location within the X-ray tube at which X-rays are generated (the X-ray focus) will change with respect to the envelope of the X-ray tube during an examination, because the mean temperature of the rotary anode may become as high as approximately 1500°C during an examination. An examination may have a duration of up to 30 seconds; the shape and dimensions of the rotary anode and of the support thereof can change due to thermal expansion during this time. Vibration can also cause movement of the X-ray focus in an X-ray source. Due to the movement of the X-ray focus in the X-ray source, a varying amount of radiation will be passed by the collimator via the space between adjacent collimator plates, because the collimator plates of the scattered radiation collimator are directed towards a predetermined location of the X-ray source. Accordingly, an apparent change will occur in the sensitivity of the detector situated behind the collimator.

The invention has for its object to provide a device of the described kind in which displacement of the X-ray focus in the X-ray source leads to a smaller apparent change in the detector sensitivity. To achieve this, a device in accordance with the invention is characterised in that at right angles to the plane of the flat, fan-shaped X-ray beam and along the line of projection of each collimating element towards the X-ray source, there is arranged a corresponding radiation absorbing element whose dimension, measured in the propagation direction of X-rays from the X-ray source, is less than that of said collimating element and whose dimension, measured in a direction transverse to said propagation direction which lies in the plane of the fan-shaped radiation beam, is greater than that of said collimating element. The amount of radiation passing between adjacent collimating elements is thus determined by the gap between adjacent absorbing elements respectively arranged in front of the corresponding collimating elements. Because the dimension of the absorbing elements measured in the direction of the X-ray source is less than the corresponding length of the collimating elements, the amount of radiation passing between adjacent collimating elements will change much less in relation to movements of the X-ray focus in the X-ray source, as will the apparent sensitivity of the X-ray detector arranged behind the collimator.

The invention will be described hereinafter, by way of example, with reference to the accompanying diagrammatic drawing.

Fig. 1 shows a device for determining local absorption differences in an object in accordance with the invention,

Fig. 2 is a sectional view taken along the line II—II in Fig. 1,

Fig. 3 is a sectional view taken along the line III—III in Fig. 2, and

Figs. 4 and 5 illustrate the operation of the device in accordance with the invention.

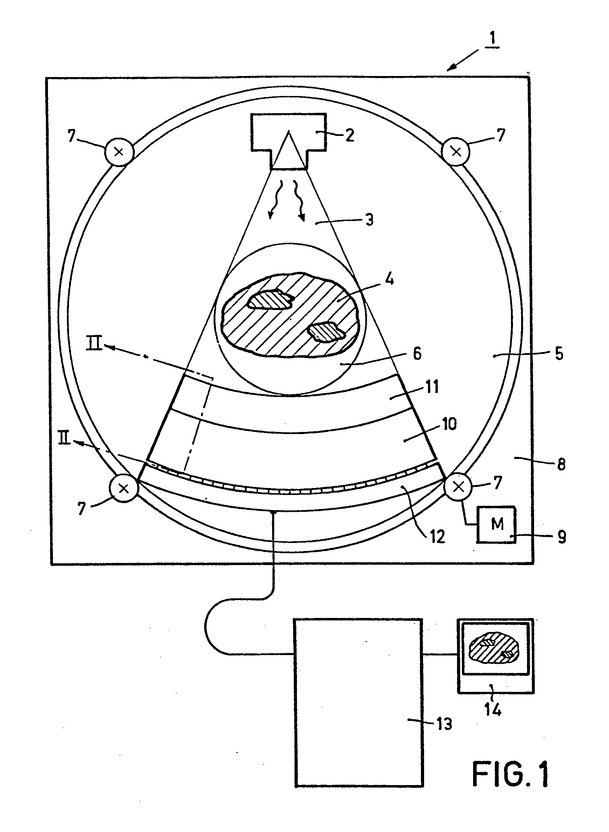

Fig. 1 diagrammatically shows a device 1 for determining local absorption differences in an object section, comprising an X-ray source 2 for generating a flat, fan-shaped X-ray beam 3 whereby an object 4 is irradiated from different directions during an examination. To achieve this, the X-ray source 2 is mounted on a rotatable support 5 which has a central aperture 6 for accommodating the object 4 and which is rotatably journalled in a frame 8 on wheels 7. During an examination, the support 5 is rotated about the central aperture 6 by means of a motor 9. On the support 5 there are also mounted so as to be always directed towards the X-ray source, an X-ray detector 10 and a scattered radiation collimator 11 which is connected to the X-ray detector. The X-ray detector 10 measures radiation transmitted by the object in the various directions, the scattered radiation collimator 11 preventing radiation which is scattered by the object — and which could have an adverse effect on the accuracy of the detector measurement signals — from reaching the X-ray detector 10. The X-ray detector 10 is connected to a signal processing circuit 12 in which detector output signals are processed to form computer input signals. Subsequently, the computer 13 calculates the density distribution in the object section 4 during an examination, said distribution being displayed on a television monitor 14 in order to be studied.

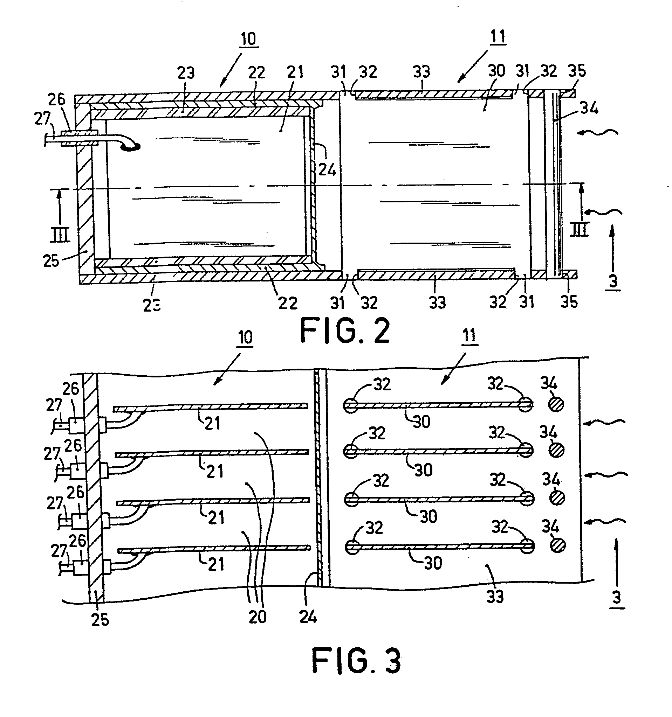

Figs. 2 and 3 are different sectional views of the X-ray detector 10 and the scattered radiation collimator 11. The X-ray detector 10 comprises an array of individual elemented ionisation chambers 20 which are arranged along an arc of a circle and which are individually defined and separated by lamina shaped electrodes 21 which are directed towards the X-ray source 2 in Fig. 1. Because said circle has a large radius in comparison with the dimensions of the electrodes 21, no curvature of the X-ray detector 10 is shown in Fig. 3. The X-ray detector furthermore comprises a housing 22 in which the electrodes 21 are mounted by means of holders 23 of a synthetic material. The housing 22 comprises an X-ray transmissive wall 24 and a rear wall 25 through which connection wires 27 for the electrodes 21 are passed via gastight seals 26. On a line of projection from each of the electrodes 21 towards the source, there is arranged a corresponding collimator plate 30 which is also directed towards the X-ray source 2, shown in Fig. 1. The collimator plates 30 form the scattered radiation collimator 11 and are secured, by means of projections 31, located in holes 32 which are respectively provided in two mounting plates 33 connected to the housing 22 of the X-ray detector 10. The collimator plates 30 are made of a material which strongly absorbs X-rays, such as W or Mo, and prevent X-rays scattered by the object 4 (in Fig. 1), from penetrating into the ionisation chambers 20.

At right angles to the plane of the flat, fan-shaped X-ray beam 3 and on respective lines of projection through the electrodes 21 and the collimator plates 30 towards the source, there are arranged X-ray absorbing elements 34. In the propagation direction of the X-rays 3 to be measured, the dimensions of the elements 34 are much less than the length of the collimator plates 30, and in a direction perpendicular to the collimator plates 30, they are much greater than the thickness of the collimator plates 30. For ease and accuracy of manufacture, the elements 34 preferably have a cylindrical shape and are made of W or Mo. The elements 34 are mounted in holes 35 in the mounting plates 33. The mounting plates 33 preferably extend parallel to one another, because the holes 32 and 35 can then be provided in one drilling operation, after which the collimator plates 30 and the elements 34 can respectively be directed accurately parallel to one another and perpendicularly to the plane of the X-ray beam 3.

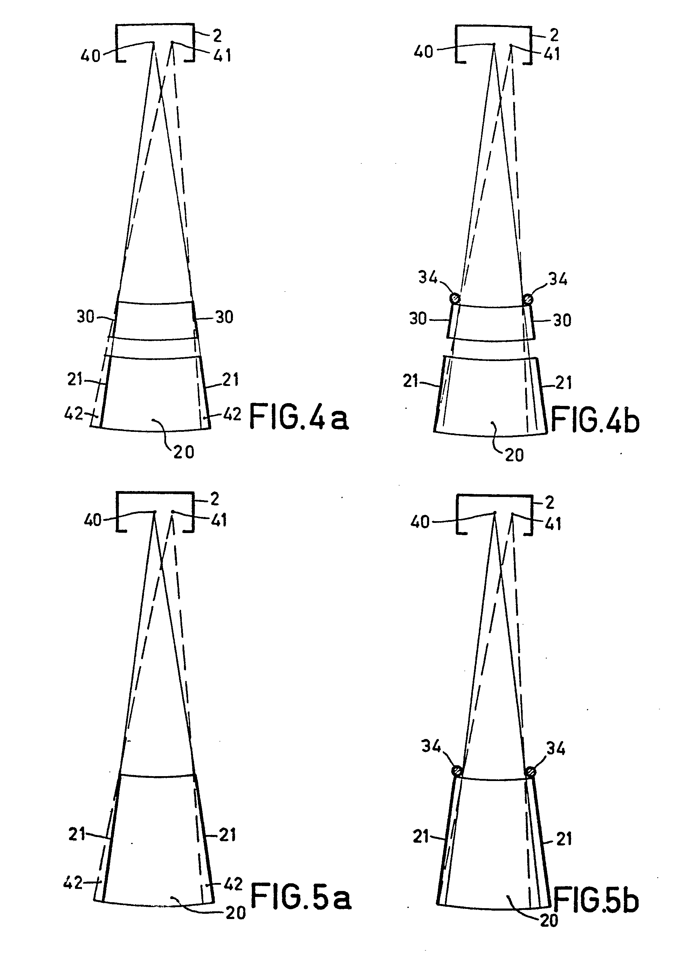

Fig. 4 diagrammatically illustrates the operation of the device in accordance with the invention and shows the X-ray source 2 and one pair of adjacent electrodes 21 of the detector, which are directed towards the X-ray source 2, collimator plates 30 and, in Fig. 4b, also one set of adjacent cylindrical elements 34. During an examination, which may last up to 30 seconds in practice, the location within the X-ray source 2 at which the X-rays are generated, namely the X-ray focus 40, is liable to shift with respect to the body of the X-ray source 2 and hence also with respect to the electrodes 21 of the detector, the collimator plates 30 and the absorbing elements 34, for example, the position 41. This shift may be caused not only by thermal expansion within the X-ray source 2, but also, for example, by mechanical vibration. Fig. 4a shows that, in the absence of the elements 34, a varying quantity of radiation will pass between the collimator plates 30 and be measured in the ionisation chamber 20 in response to the shift of the X-ray focus within the X-ray source 2. The collimator plates 30 cast their shadow 42, as it were, into the ionisation chamber 20. This problem is solved by using the radiation absorbing elements 34. The amount of radiation which then passes between the adjacent collimator plates 30 will be determined by the gap between the elements 34 and, because the dimension thereof in the direction of the corresponding collimator plate 30 is much less than the length of the collimator plate 30, the amount of radiation passing between the collimator plates 30 in response to the shift from 40 to 41 of the X-ray focus within the X-ray source 2, will vary to a much smaller extent. Thus the apparent change in the sensitivity of the X-ray detector 10, giving rise to disturbing errors in the calculation of the density distribution of the object 4, will thus be substantially mitigated.

The foregoing description relates to a device 1 comprising a detector 10 with a separate collimator 11 comprising a number of radiation absorbing collimator plates 30 directed towards the X-ray source 2. If the lamina shaped electrodes 21 of the X-ray detector 10 themselves contain a radiation-absorbing material, however, these electrodes 21 themselves will act as a collimating element and the collimator plates 30 can possibly be dispensed with, as shown in the Figs. 5a and 5b. A shift from 40 to 41 of the X-ray focus within the X-ray source 2 will again cause a varying amount of radiation to be measured, as shown in Fig. 5a. This problem is again solved by using radiation absorbing elements 34 as will be apparent from Fig. 5b.

## Claims

1. A device for determining local absorption differences in an object section, comprising an X-ray source (2) for generating a flat, fan-shaped X-ray beam (3) and an X-ray detector (10) in the general form of an arc of a circle having as its centre the X-ray source, the X-ray detector being directed radially towards the X-ray source and including a plurality of lamina-shaped collimating elements (30) which are also directed radially towards the X-ray source, characterized in that at right angles to the plane of the flat, fan-shaped X-ray beam and along the line of projection of each collimating element towards the X-ray source, there is arranged a corresponding radiation absorbing element (34) whose dimension, measured in the propagation direction of X-rays from the X-ray source is less than that of said collimating element (30) and whose dimension, measured in a direction transverse to said propagation direction which lies in the plane of the fan-shaped radiation beam, is greater than that of said collimating element.

2. A device as claimed in Claim 1, characterized in that the absorbing elements (30) are cylindrical in form.

3. A device as claimed in Claim 1 or 2, characterized in that the absorbing elements (30) are made of W or Mo.

4. A device as claimed in Claim 2, characterized in that the cylindrical absorbing elements (30) are arranged between two parallel mounting plates (33) which are directed at right angles to the collimator plates.

## Patentansprüche

1. Vorrichtung zum Ermitteln örtlicher Absorptionsunterschiede in einem Teil eines Objeks, mit einer Röntgenstrahlungsquelle (2) zum Erzeugen eines flachen, fächerförmigen Röntgenstrahlenbündels (3) und mit einem Röntgendetektor (10) in allgemeiner Bogenform oder Kreisform, wobei die Mitte von der Röntgenstrahlungsquelle gebildet, wird, wobei der Röntgendetektor radial auf die Röntgenstrahlungsquelle gerichtet ist und eine Vielzahl laminierter Kollimierelemente (30) enthält, die ebenfalls radial auf die Röntgenstrahlungsquelle ausgerichtet sind, dadurch gekennzeichnet, dass senkrecht zur Ebene des flachen, fächerförmigen Röntgenstrahlungsbündels und entlang der Projektionslinie eines jeden Kollimierelements zur Röntgenstrahlungsquelle ein entsprechendes, Strahlung absorbierendes Element (34) vorgesehen ist, dessen Abmessung, gemessen in der Fortpflanzungsrichtung der Röntgenstrahlen aus der Röntgenstrahlungsquelle, kleiner als die des genannten Kollimierelements (30) ist und dessen Abmessung, gemessen in einer Richtung quer zur Fortpflanzungsrichtung, die in der Ebene des fächerförmigen Röntgenstrahlungsbündels liegt, grösser als die des genannten Kollimierelements ist.

2. Vorrichtung nach Anspruch 1, dadurch gekennzeichnet, dass die absorbierenden Elemente (34) Zylinderform haben.

3. Vorrichtung nach Anspruch 1 oder 2, dadurch gekennzeichnet, dass die absorbierenden Elemente (34) aus W oder aus Mo bestehen.

4. Vorrichtung nach Anspruch 2, dadurch gekennzeichnet, dass die zylindrischen absorbierenden Elemente (34) zwischen zwei parallel verlaufenden Montageplatten (33) angeordnet sind, die senkrecht zu den Killimatorplatten verlaufen.

## Revendications

1. Dispositif pour déterminer les différences locales d'absorption dans une section d'objet comprenant une source (2) de rayons X destinée à produire un faisceau (3) de rayons X plat en forme d'éventail et un détecteur (10) de rayons X ayant d'une manière générale la forme d'un arc de cercle dont le centre es constitué par la source de rayons X, le détecteur de rayons X étant dirigé radialement vers la source de rayons X et comprenant plusieurs éléments collimateurs en forme de lames (30) qui sont également dirigés radialement vers la source de rayons X, caractérisé en ce que perpendiculairement au plan du faisceau de rayons X plat en forme d'éventail et le long de la ligne de projection de chaque élément collimateur vers la source de rayons X est disposé un élément absorbant la rayonnement (34) correspondant dont la dimension mesurée dans la direction de propagation des rayons X à partir de la source de rayons X est inférieure à celle de l'élément collimateur (30) et dont la dimension mesurée dans une direction transversale à la direction de propagation, qui est située dans le plan du faisceau de rayonnement en forme d'éventail, est supérieure à celle de l'élément collimateur.

2. Dispositif suivant la revendication 1, caractérisé en ce que les éléments absorbants (30) sont de forme cylindrique.

3. Dispositif suivant la revendication 1 ou 2, caractérisé en ce que les éléments absorbants (30) sont en W ou en Mo.

4. Dispositif suivant la revendication 2, caractérisé en ce que les éléments absorbants cylindriques (30) sont disposés entre deux plaques de montage parallèles (33) qui sont orientées perpendiculairement aux plaques collimatrices.

FIG.1

FIG. 2

FIG. 3

FIG.4a

FIG.4b

FIG.5a

FIG.5b